# FASCICULE DE BREVET EUROPEEN

(11) **EP 0 597 747 B1**
(45) Date de publication et mention de la délivrance du brevet: **07.02.1996**
(21) Numéro de dépôt: 93402607.1
(22) Date de dépôt: 25.10.1993
(51) Int. Cl.: C07C 321/18, C07C 323/27, C07C 323/54, C08F 2/38, C08F 112/08, C08F 120/14

(54) **Utilisation de diènes comme agent de transfert lors de la préparation de polymères**
Verwendung von Dienen als Übertragungsmittel während der Herstellung von Polymeren
Use of dienes as transfer agent during the preparation of polymers

(30) Priorité: 10.11.1992 FR 9213520
(43) Date de publication de la demande: 18.05.1994
(73) Titulaire: RHONE-POULENC CHIMIE, F-92408 Courbevoie Cédex (FR)
(72) Inventeur: Charmot, Dominique, F-93310 Le Pré Saint Gervais (FR); Oger, Nicole, F-92000 Nanterre (FR); Viehe, Heinz, B-1300 Limal (BE)
(74) Mandataire: Seugnet, Jean Louis

(56) Documents cités:
- EP-A- 0 130 637
- EP-A- 0 469 954
- WO-A-88/04304
- CHEMICAL ABSTRACTS, vol. 79, no. 11, 17 Septembre 1973, Columbus, Ohio, US; abstract no. 65709p, *
- CHEMICAL ABSTRACTS, vol. 98, no. 19, 9 Mai 1983, Columbus, Ohio, US; abstract no. 159844y, *
- TETRAHEDRON LETTERS vol. 30, no. 46, 1989, OXFORD GB pages 6405 - 6408 TATSUO HAMADA ET AL. 'Diels-Alder reaction of chiral dienes. Remarkable effect of dienophile polarity upon diastereo face selectivity' *

## Description

La présente invention concerne un procédé de préparation de polymères par polymérisation de monomères éthyléniquement insaturés en présence d'un diène conjugué comme agent de transfert de chaînes (ou limitateur de chaînes).

Pour imiter le poids moléculaire et/ou le degré de réticulation du polymère formé, qui ont des influences profondes sur les propriétés d'usage , il est usuel d'employer un agent de transfert de chaîne (dénommé également agent limitateur de chaîne) qui agit au cours du processus de polymérisation. Les agents de transfert couramment utilisés industriellement comprennent les mercaptans aliphatiques et les hydrocarbures halogénés. Ces deux types de régulateurs présentent des inconvénients. Les polymères qui ont été obtenus en présence d'un mercaptan, bien qu'ayant une teneur résiduelle en mercaptan très faible, présentent une odeur caractéristique indésirable. Si l'on utilise des composés organohalogénés (voir par exemple US-A 4.176.219), comme le tétrachlorure de carbone, le bromoforme ou le bromotrichlorométhane, les teneurs résiduelles sont relativement élevées.

US-A 3.726.832 décrit la préparation de polymères de dioléfines conjuguées ayant un poids moléculaire inférieur à un million, à une vitesse de polymérisation élevée, avec mise en oeuvre d'éthers cycliques servant d'agent de régulation du poids moléculaire.

WO-A88/04.304 décrit un agent de transfert de chaîne choisi parmi les produits de formule : 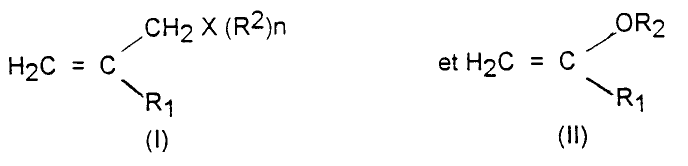
R₁ étant de préférence un groupe capable d'activer la liaison éthylénique tel qu'un groupe araomatique, R₂ est notamment un groupe alkyle et X est un hétéroatome.

D'après ce brevet, les agents de transfert de chaîne de formule (I) et (II) présentent une constante de transfert de chaîne voisine de 1 ce qui permet d'obtenir des polymères ayant une distribution moléculaire en poids étroite.

La présente invention a pour but de remédier aux inconvénients de l'art antérieur en proposant un procédé de polymérisation efficace, résultant en une bonne conversion des monomères, qui conduit à des polymères ou copolymères ne présentant pas ou peu d'odeur indésirable ni de composés résiduels halogénés sans nécessiter un traitement de post-polymérisation ou élimination de l'agent de transfert résiduel.

Elle a pour but également de proposer des agents de transfert présentant une constante de transfert de chaîne voisine de 1 et qui conduisent à des polymères ayant la longueur de chaîne désirée et fonctionnalisés en bout de chaînes par un diène conjugué.

Ces buts et d'autres sont atteints par la présente invention qui concerne en effet de nouveaux agents de transfert qui présentent les avantages mentionnés ci-dessus et qui ne présentent pas les inconvénients des produits de l'art antérieur ou alors sous une forme très atténée.

Les diènes conjugués selon l'invention sont des produits nouveaux et répondent à la formule générale : 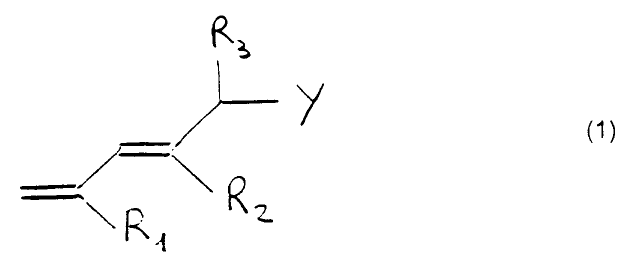
dans laquelle;
. les radicaux R₁, R₂, et R₃ identiques ou différents sont choisis parmi un atome d'hydrogène, un radical alkyle linéaire ou ramifié en C₁ - C₆, un radical phényle, un radical alcoxycarbonyle, un radical acyloxy et un radical acylamino, dans lesquels la partie alkyle est linéaire, cyclique ou ramifiée en C₁ - C₆, un radical phénoxycarbonyle et un radical cyano,
. le radical Y est choisi parmi les radicaux :
   X - R4 et - C H(R5 R6) dans lesquels :
   R4 est choisi parmi un radical alkyle linéaire ou ramifié en C₁ - C₆, un radical phényle et un radical acyle dont la partie alkyle linéaire ou ramifiée est en C₁ - C₆
   X est un hétéroatome choisi parmi O et S.
   R5 est un radical capteur d'électrons choisi parmi un radical cyano, carbamoyle, phényloxycarbonyle, alcoxycarbonyle et phénylcarbonyle dans lequel la partie alkyle linéaire, cyclique ou ramifiée est en C₁ - C₆,
   R6 est un radical donneur d'électron choisi parmi un radical alkylthyoéther, phénylthioéther, amino, alkylamino, alcoxy et phénoxy dans lequel la partie alkyle linéaire, cyclique ou ramifiée est en C₁ - C₆

Comme exemple de diène répondant à la formule (1), on peut citer ceux de formule : 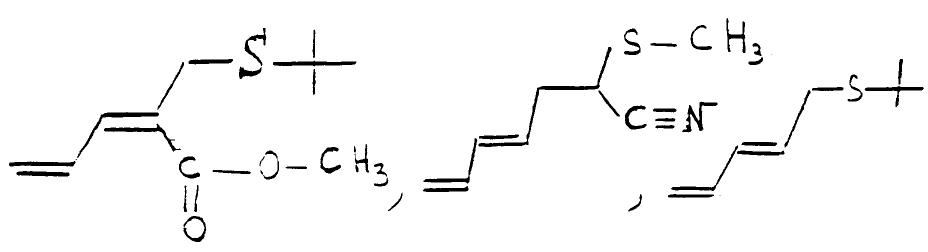
La présente invention concerne également un procédé de préparation de polymères selon lequel on polymérise, de préférence en émulsion aqueuse, au moins un monomère à insaturation éthylénique par polymérisation radicalaire en présence d'une quantité efficace d'un agent de transfert répondant à la formule 1. Comme monomère à insaturation éthylénique, on vise plus spécifiquement selon l'invention les monomères choisis parmi le styrène, le butadiène, les esters (métha)acryliques et les nitriles vinyliques. Par esters (méth)acryliques, on désigne les esters de l'acide acrylique et de l'acide méthacrylique avec les alcools en C₁-C₁₂, de préférence C₁-C₈, tels que l'acrylate de méthyle, l'acrylate d'éthyle, l'acrylate de propyle, l'acrylate de n-butyle, l'acrylate d'isobutyle, l'acrylate de 2-éthylhexyle, le méthacrylate de méthyle, le méthacrylate d'éthyle, le méthacrylate de n-butyle, le méthacrylate d'isobutyle... Les nitriles vinyliques incluent ceux ayant de 3 à 12 atomes de carbone, en particulier l'acrylonitrile et le méthacrylonitrile.
Le styrène peut être remplacé en totalité ou en partie par l'alphaméthylstyrène ou le vinyltoluène.

Plus particulièrement les monomères à insaturation éthylénique à polymériser sont constitués par au moins 60 % en poids d'au moins un monomère cité ci-dessus en mélange avec jusqu'à 40 % en poids d'au moins un autre monomère éthyléniquement insaturé copolymérisable.

Parmi les monomères éthyléniquement insaturés copolymérisables avec les monomères ci-dessus, et dont la quantité peut aller jusquà 40 % en poids du total des monomères on peut citer :
- les esters vinyliques d'acide carboxylique comme l'acétate de vinyle, le versatate de vinyle, le propionate de vinyle,
- les acides mono-et di- carboxyliques insaturés éthyléniques comme l'acide acrylique, l'acide méthacrylique, l'acide itaconique, l'acide maléique, l'acide fumarique et les mono-alkylesters des acides dicarboxyliques du type cité avec les alcanols ayant de préférence 1 à 4 atomes de carbone et leurs dérivés N-substitués,
- les amides des acides carboxyliques insaturés comme l'acrylamide, le méthacrylamide, le N-méthylolacrylamide ou méthacrylamide,
- les monomères éthyléniques comportant un groupe acide sulfonique et ses sels alcalins ou d'ammonium par exemple l'acide vinylsulfonique, l'acide vinylbenzène sulfonique, l'acide α-acrylamido méthylpropane-sulfonique, le 2-sulfoéthylène-méthacrylate,
- les monomères éthyléniques insaturés comportant un groupe aminosecondaire, tertiaire ou quaternaire ou un groupe hétérocyclique contenant de l'azote par exemple les vinylpyridines, le vinylimidazole, les (meth)acrylates d'aminoalkyle et les (meth)acrylamides d'aminoalkyle comme le diméthylaminoéthylacrylate ou -méthacrylate, le ditertiobutylaminoéthylacrylate ou -méthacrylate, le diméthylamino méthylacrylamide ou -méthacrylamide...de même que les monomères zwitterioniques comme l'acrylate de sulfopropyl (diméthyl)aminopropyle...
- les esters des acides (meth)acryliques avec des alcanediols contenant de préférence 2 - 8 atomes de carbone tels que le mono (meth)acrylate de glycol, le mono(meth)acrylate d'hydroxypropyle, le mono (meth)acrylate de 1-4 butanediol ainsi que les monomères comportant deux doubles liaisons polymérisables comme le diméthacrylate d'éthylène-glycol.

La polymérisation est réalisée de manière connue en soi, en solution, en masse, en émulsion aqueuse en présence d'au moins un initiateur radicalaire et de l'agent de transfert avec une concentration en monomères dans le milieu réactionnel comprise généralement entre 20 et 60 % en poids.

Tout type d'initiateur ou amorceur à radicaux libres habituels dans la polymérisation radicalaire peut convenir. Des exemples d'initiateurs comprennent les hydroperoxydes tels que l'eau oxygénée, l'hydroperoxyde de diisopropylbenzène, les persulfates de sodium, de potassium ou d'ammonium, et les initiateurs cationiques comme l'azobis(isobutyronitrile) le 4-4'azobis (acide 4-cyano valérique).

Ces initiateurs peuvent être associés à un réducteur comme par exemple le bisulfite. La quantité se situe en général entre 0,05 et 2 % en poids par rapport à la quantité des monomères.

La quantité de diène de formule 1 est généralement comprise entre 0,05 et 10 %, de préférence entre 0,1 et 3 % en poids par rapport au poids total des monomères, suivant le poids moléculaire et le degré de réticulation souhaité pour le polymère. Le diène peut être introduit dans le milieu réactionnel soit en totalité au début de la réaction soit en continu en solution dans les monomères principaux, soit encore en partie initialement et en partie en continu. Lorsque la solubilité du produit dans les monomères est faible, il peut être introduit sous forme d'une suspension simultanément avec les monomères.

La température de polymérisation, fonction de l'initiateur mis en oeuvre, est généralement comprise entre 50°C et 100°C, de préférence entre 70 et 90°C. Dans le cas où la polymérisation est éffectuée en émulsion aqueuse, la stabilisation des particules est assurée, si nécessaire, par tout système de stabilisation colloïdale connu comme les émulsifiants anioniques, cationiques, amphotères et non ioniques. La polymérisation peut être réalisée en continu, en discontinu ou semi-continu avec introduction d'une partie des monomères en continu et être du type "ensemencé" ou "incremental" selon toute variante connue pour l'obtention des particules de structure homogène et hétérogène.

Les polymères et les copolymères inodores préparés selon le procédé de l'invention se caractérisent en ce quils contiennent en bout de chaîne des diènes conjugués provenant de l'agent de transfert selon l'invention. Ces diènes conjugués en extrémité de chaînes peuvent être utilisables par exemple pour une copolymérisation ou une réticulation ultérieure ou la synthèse de copolymères blocs.

Les solutions dans un solvant organique ou les dispersions aqueuses de polymères peuvent être avantageusement utilisées comme liants dans les compositions de revêtement pour le couchage du papier, comme liants dans l'industrie textile en particulier pour la fabrication de non tissés, comme additif dans les peintures et les formulations adhésives.

Les exemples qui suivent, dans lesquels les parties et les pourcentages sont indiqués en poids, illustrent l'invention.

### EXEMPLE 1 :

### Préparation de l'agent de transfert de formule :

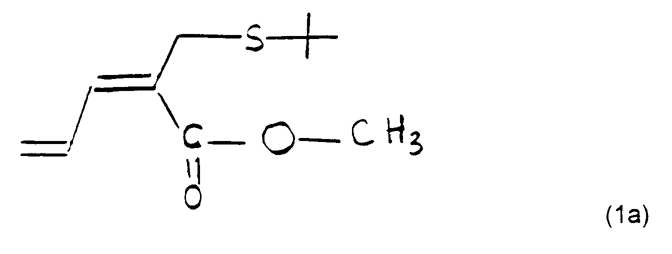

Le produit de formule (1a) est obtenu par réaction du tertio butylthiol sur le 1,3-pentanediénoïque acide 2-méthyl 4-(bromo méthyl) méthyl ester. Lui-même est obtenu par bromation par PBr₃ du 1,4-butadiène 2-méthyl 3-hydroxy 4-méthoxycarbonyl, lui-même obtenu par condensation de l'acrylate de méthyle sur la méthacroléine.
Le mélange de 13 g (0,15 mole) d'acrylate de méthyle, de 7 g (0,1 mole) de méthacroléine et de 1,6 g (0,015 mole) de DABCO est agité à température ambiante pendant 21 jours. Les produits de départ qui n'ont pas réagi sont éliminés au rotavapor. Le résidu est mis en solution dans 50 ml d'éther et lavé avec 100 ml d'une solution d'acide chlorhydrique à 10 %, puis deux fois avec 100 ml d'eau. Après évaporation de l'éther, 10,1 g (rendement : 65 %) de liquide (1,4 butadiène 2- méthyl 3-hydroxy 4-méthoxycarbonyl) sont obtenus.
A une solution de 1,4 -butadiène 2-méthyl 3-hydroxy 4-méthoxycarbonyl (7,7 g ; 0,05 mole) dans l'éther (100 ml) maintenue à - 10°C, est ajoutée une solution de PBr₃ (10 g, 0,037 mole) dans l'éther. La réaction est exothermique avec dégagement de HBr. La solution est agitée et maintenue à température ambiante pendant trois heures. La température est abaissée à - 10°C et 100 ml d'eau permutée sont ajoutés. Le mélange est extrait à l'hexane (trois fois 20 ml) et la phase organique est lavée deux fois avec une solution saturée de chlorure de sodium et séchée sur MgSO₄. Le solvant est évaporé et un liquide (1,3-pentadiénoïque acide 2-méthyl 4-(bromométhyl) méthylester est obtenu (10,5 g ; rendement : 96 %).

1,37 g de carbonate de potassium (0,01 mole), 2,19 g de (1,3-pentadiénoïque acide 2-méthyl 4-(bromométhyl)méthylester (0,01 mole), 0,9 g de t-butyl thiol (0,01 mole) et 50 ml de méthanol sont agités pendant 20 heures. Le méthanol est évaporé au rotavapor et 20 ml d'eau sont ajoutés. La phase aqueuse est lavée avec trois fois 20 ml d'éther et les phases organiques sont rassemblées et séchées sur MgSO₄. Le solvant est évaporé et le produit est distillé sous pression réduite , 2 g de produit de formule (1a) sont obtenus (rendement pondéral: 87 %).

### EXEMPLE 2 :

### Préparation de polystyrène en présence de l'agent de transfert de formule (1a) :

70 mg de Azo-bis-isobutyronitrile (AIBN) sont introduits dans 45 g de styrène fraîchement distillé, 4,5 g du mélange sont introduits dans un ballon de 100 ml contenant la quantité indiquée au tableau 1 ci-après de produit de formule (1a). Le mélange est polymérisé 1 heure à 60°C en l'absence d'oxygène, puis précipité par un excès de méthanol. Le polymère est ensuite collecté et séché jusqu'à poids constant. Le polymère est ensuite examiné par GPC (Chromatographie par Perméation de gel), le tétrahydrofurane étant utilisé comme éluant à un débit de 1 ml/mn ; l'appareillage étant constitué d'une pompe WATERS ® connectée avec 4 colonnes PLGEL ® (taille des pores : 10⁵ et 50 nm) et deux colonnes mixtes dont les tailles des pores varient entre 50 et 10⁻³ nm et d'un réfractomètre différentiel. Le système est câlibré à l'aide de polystyrène-étalon de masse moléculaire connue et de faible polydispersité.

Les résultats obtenus sont rassemblés dans le tableau 1 ci-après :

**TABLEAU 1**

| **Quantité d'agent de transfert (1a)(mg)** | **Conversion (-%)** | **Mn* (x 1000)** |
|---|---|---|
| 0 | 2,6 | 103 |
| 49 | 0,55 | 11 |
| Mn* : masse moléculaire moyenne en nombre en g/mol. | | |

Les résultats rassemblés dans le tableau 1 indiquent que le produit de formule (1a) est un très bon agent de transfert.

### EXEMPLE 3 :

### Préparation de polyméthacrylate de méthyle en présence de l'agent de transfert de formule (1a) :

On procède exactement comme dans l'exemple 2 ci-dessus sauf qu'on remplace le styrène par le méthacrylate de méthyle et qu'on utilise des quantités d'agent de transfert indiquées au tableau 2 ci-après.

**TABLEAU 2**

| **Quantité d'agent de transfert (1a) (mg)** | **Conversion (%)** | **Mn* (x 1000)** |
|---|---|---|
| 0 | 2,8 | 201 |
| 13 | 0,7 | 48 |
| Mn* : masse moléculaire moyenne en nombre en équivalent polystyrène en g/mol. | | |

Les résultats rassemblés dans le tableau 2 indiquent que le produit de formule (1a) est un très bon agent de transfert.

### EXEMPLE 4 :

### Préparation de l'agent de transfert de formule :

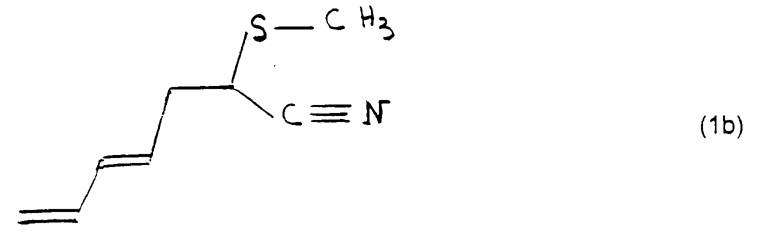

Le produit de formule (1b) est obtenu par bromation du 1,4-pentadiène 3-ol, suivie de la réaction du méthylthio acétonitrile sur le 5-bromo 1,3-pentadiène.

A une solution de 1,4-pentadiène 3-ol (4,2 g; 0,05 mole) dans l'éther (100 ml) maintenue à - 10°C, est ajoutée une solution de PBr3 (10 g; 0,037 mole) dans l'éther. La réaction est exothermique avec dégagement de HBr. La solution est agitée et maintenue à température ambiante pendant 3 heures. La température est à nouveau abaissée à - 10°C et 10 ml d'eau sont ajoutés. Le mélange est extrait à l'hexane (trois fois 20 ml) et la phase organique est lavée deux fois avec une solution saturée de NaCl et séchée sur MgSO4. Le solvant est évaporé et le 5-bromo 1,3-pentadiène est obtenu (7 g; rendement pondéral 95 %).
A une solution de 0,6 g (0,02 mole) de NaH dans le THF sec, à l'abri de l'air et de l'eau, 1,8 g (0,02 mole) de méthylthio acétonitrile sont ajoutés goutte à goutte. La solution est agitée à température ambiante pendant 15 minutes et 3 g (0,02 mole) de 5-bromo 1,3-pentadiène sont ajoutés à température ambiante. 100 ml d'eau permutée sont ajoutés goutte àgoutte et la solution est extraite avec de de l'éther (3 fois 20 ml) puis séchée sur MgSO4. Le solvant est évaporé et le produit de formule (1b) est obtenu (1,5 g; rendement 50 %)

### EXEMPLE 5 :

### Préparation de polystyrène en présence de l'agent de transfert de formule (1b) :

On procède exactement comme à l'exemple 2, en utilisant les quantités de produit de formule (1b) indiquées au tableau 3 et où les résultats sont également rassemblés.

**TABLEAU 3**

| **Quantité de produit (1b) (mg)** | **Conversion (%)** | **Mn* (x 1000)** |
|---|---|---|
| 0 | 2,6 | 103 |
| 32 | 0,17 | 10 |

### EXEMPLE 6 :

Préparation de polyméthacrylate de méthyle en présence de l'agent de transfert de formule (1b).

On procède exactement comme à l'exemple 3, en utilisant les quantités de produit de formule (1b) indiquées au tableau 4 et où les résultats sont également rassemblés.

**TABLEAU 4**

| **Quantité de produit (1b) (mg)** | **Conversion (%)** | **Mn* (x 1000)** |
|---|---|---|
| 0 | 2,8 | 201 |
| 100,2 | 0,02 | 27 |

### EXEMPLE 7 :

### Synthèse de l'agent de transfert de formule (1c) :

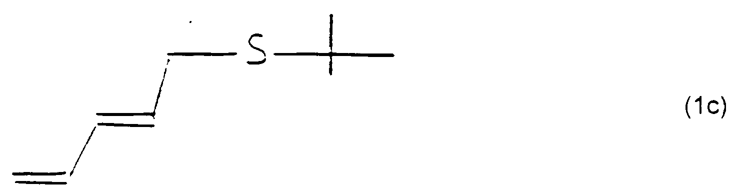

1,37 g de carbonate de potassium, (0,01 mole), 1,5 g de 5-bromo 1,3-pentadiène (0,01 mole), 0,9 g de t - bytyl thiol (0,01 mole) et 50 ml de méthanol sont agités pendant 20 heures; Le méthanol est évaporé au rotavapor et 20 ml d'eau sont ajoutés. La phase aqueuse est lavée avec trois fois 30 ml d'éther et les phases organiques sont rassemblés et séchés sur MgSO4. Le solvant est évaporé et le produit distillé sous pression réduite. On obtient 1,32 g de produit de formule (1c) avec un rendement pondéral de 87 %.

### EXEMPLE 8 :

### Préparation de polystyrène en présence de l'agent de transfert de formule (1c) :

On procède exactement comme à l'exemple 2 en utilisant les quantités de produit indiquées au tableau 5 ci- après où sont également rassemblés les résultas.

**TABLEAU 5**

| **Quantité de produit de formule (1c) (mg)** | **Conversion (%)** | **Mn* (x 1000)** |
|---|---|---|
| 0 | 2,6 | 103 |
| 29,8 | 2,02 | 29 |

### EXEMPLE 9 :

### Préparation de polyméthacrylate de méthyle en présence de l'agent de transfert deformule (1c):

On procéde exactement comme dans l'exemple 3, en utilisant les quantités indiquées dans le tableau 6 ci-dessous où sont également rassemblés les résultats.

**TABLEAU 6**

| **Quantité de produit (1c) (mg)** | **Conversion (%)** | **Mn* (x 1000)** |
|---|---|---|
| 0 | 2,6 | 103 |
| 34,7 | 0,3 | 20 |

### EXEMPLE 10 :

### Préparation de polystyrène en présence de l'agent de transfert de formule (1c) :

Le styrène, l'AIBN et le produit de formule (1c) préparé selon l'exemple 7 ci-dessus sont mélangés dans un tube de verre selon les quantités indiquées tableau 7 ci-après. Le milieu réactionnel est désaéré par trois cycles de congélation et mis sous vide, puis le tube est scellé sous vide et porté à la température indiquée. Le contenu est ensuite dilué dans 150 ml de THF et le polymère précipité dans 1,5 l de méthanol.
Après filtration, le polymère obtenu est redissous dans 150 ml de THF et de nouveau précipité dans 1,5 l de méthanol. Le filtrat obtenu est séché sous vide à 50°C jusqu'à poids constant. Le polymère est ensuite examinè par GPC, selon les conditions indiquées à l'exemple 2 ci-dessus. Les résultats obtenus sont rassemblés tableau 7 ci-après.

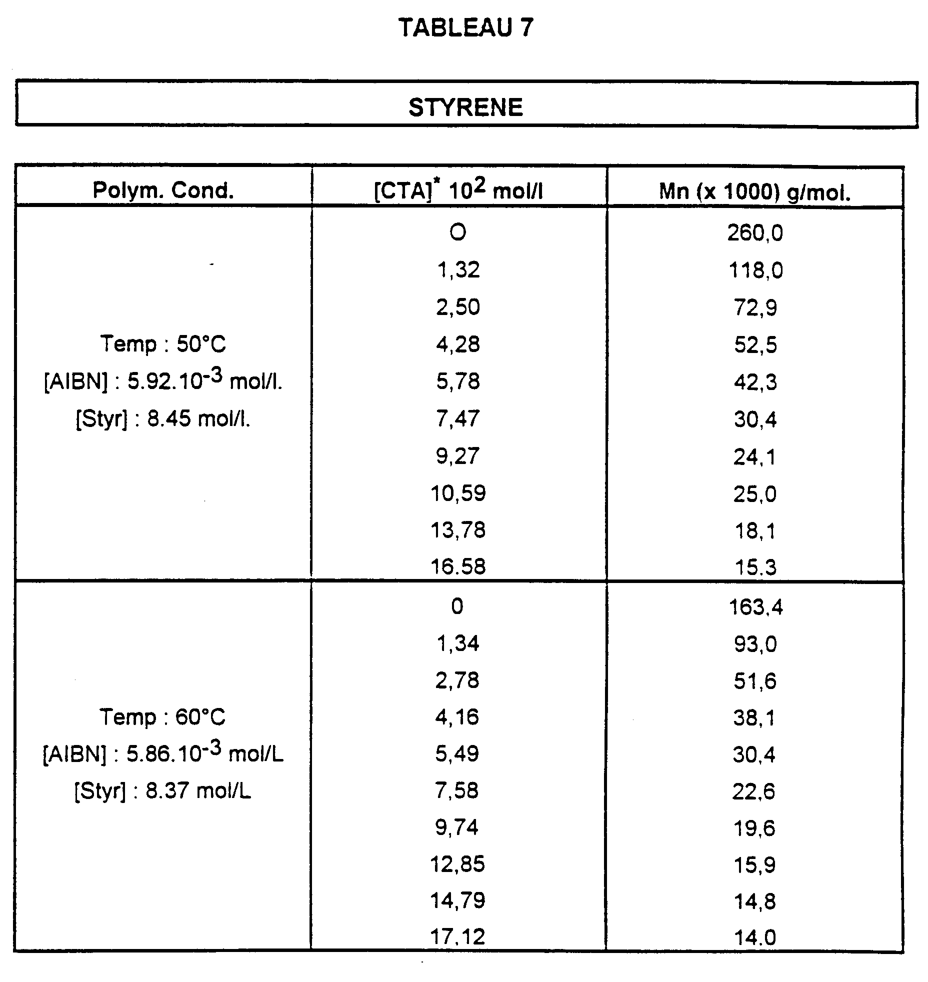

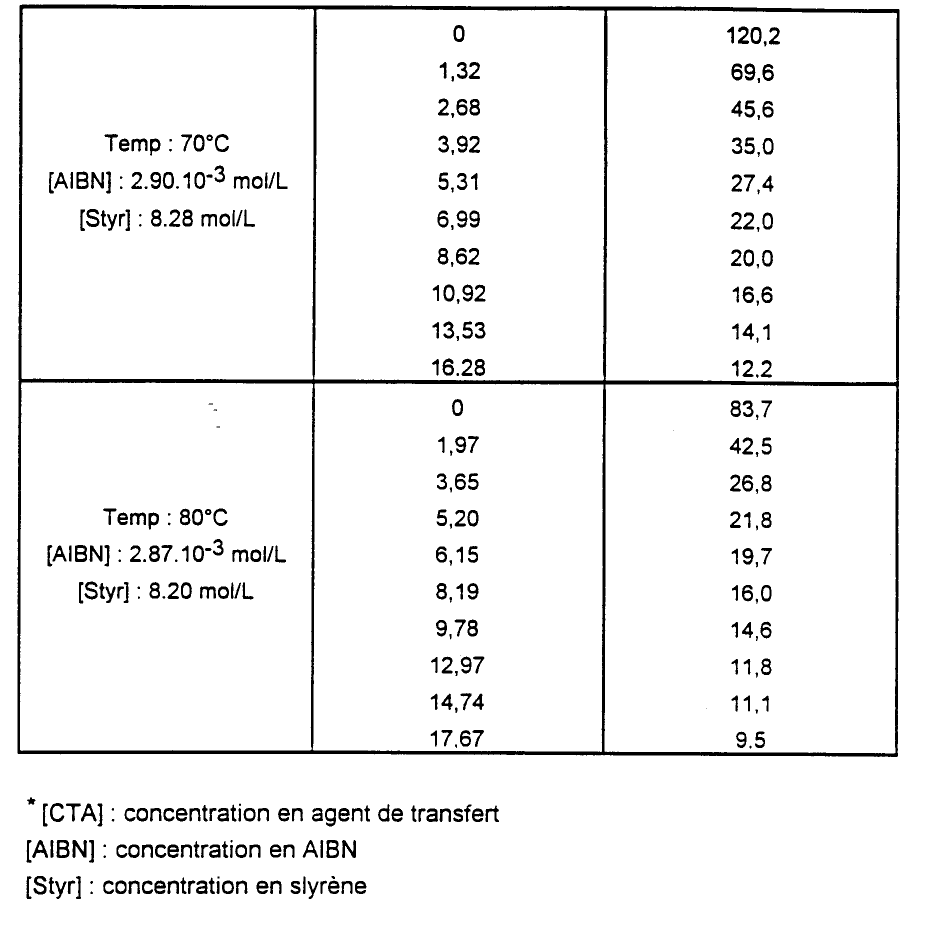

### EXEMPLE 11 :

### Préparation de polyméthacrylate de méthyle en présence de (1c)

Le méthacrylate de méthyle, l'AIBN et le produit de formule (1c) préparé selon l'exemple 7 ci-dessus sont mélangés dans un tube de verre selon les quantités indiquées tableau 8 ci-après. Le milieu réactionnel est désaéré par trois cycles de congélation et mise sous vide, puis le tube est scellé sous vide et porté à la température indiquée. Le contenu est ensuite dilué dans 150 ml de THF et le polymère précipité dans 1,5 l d'heptane.
Le produit précipité est filtré, séché à 50°C sous vide jusqu'au poids constant.

Le polymère est ensuite examinè par GPC, selon les conditionsindiquées exemple 2.

Les résultats obtenus sont rassemblés tableau 8 :

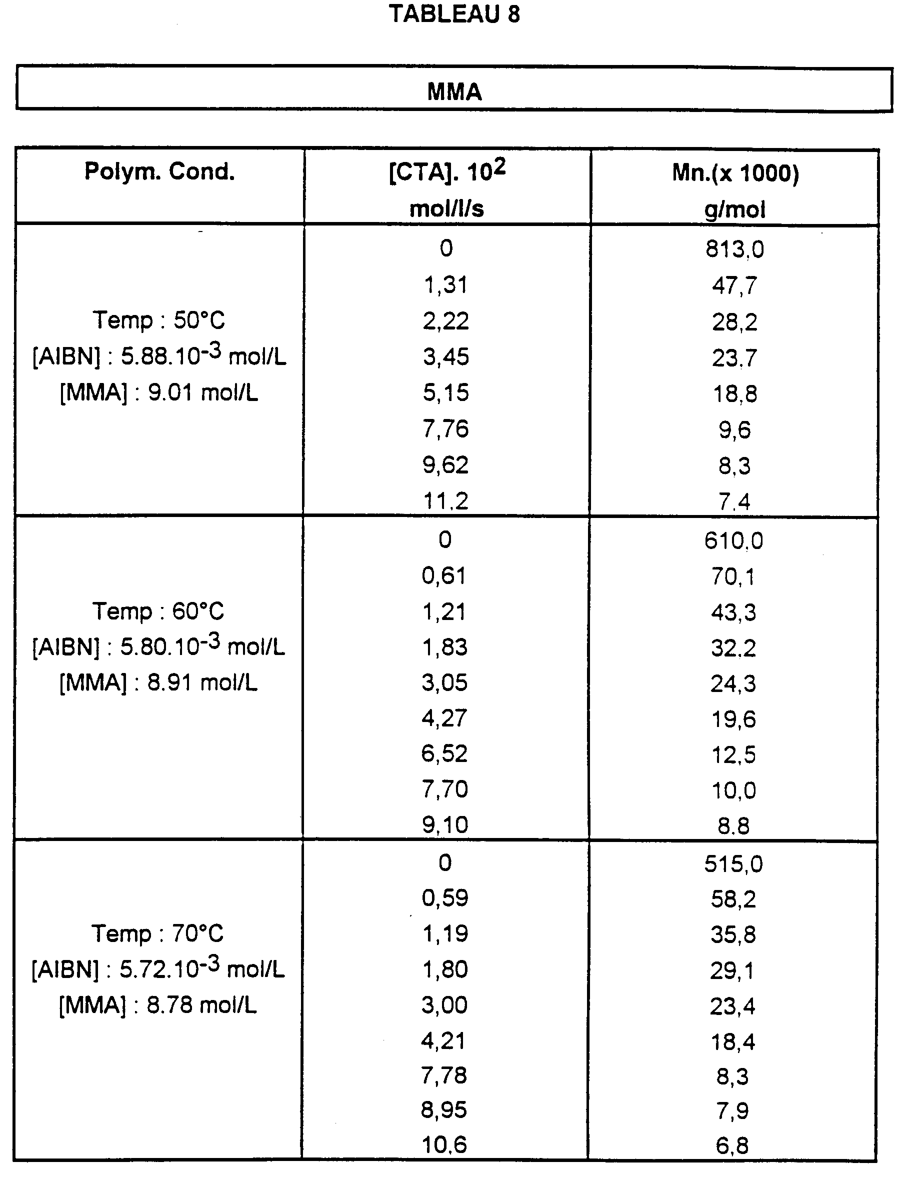

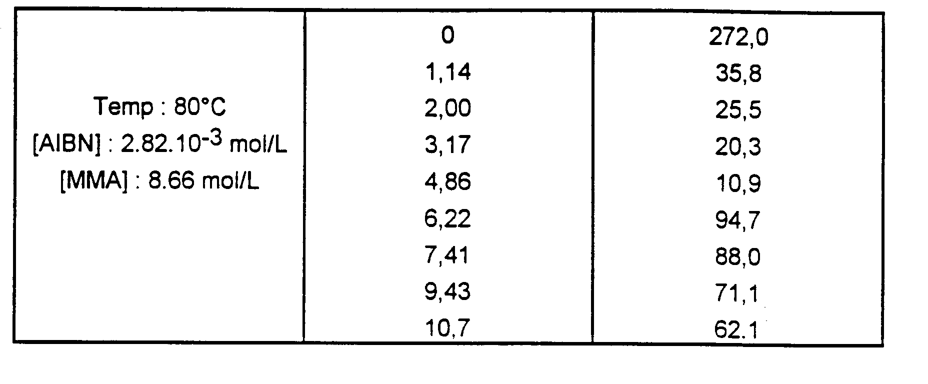

## Revendications

1. Procédé de préparation de polymère selon lequel on polymérise au moins un monomère à insaturation éthylénique par polymérisation radicalaire en présence d'une quantité efficace d'un agent de transfert diène conjugué répondant à la formule générale : 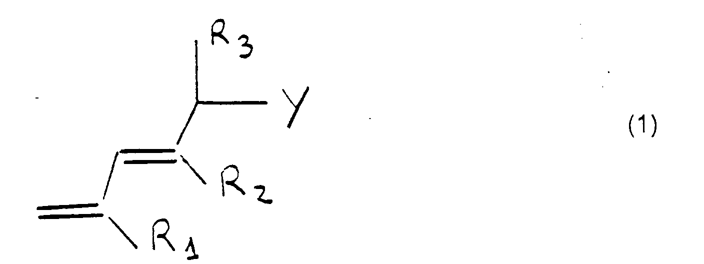 dans laquelle ;
. les radicaux R₁, R₂,et R₃ identiques ou différents sont choisis parmi un atome d'hydrogène, un radical alkyle linéaire ou ramifié en C₁ - C₆, un radical phényle, un radical alcoxycarbonyle, un radical acyloxy et un radical acylamino, dans lesquels la partie alkyle est linéaire ou ramifiée en C₁ - C₆, un radical phénoxycarbonyle et un radical cyano.
Le radical Y est choisi parmi les radicaux :
X - R4 et - C H(R5 R6) dans lesquels :
R4 est choisi parmi un radical alkyle linéaire ou ramifié en C₁ - C₆, un radical phényle et un radical acyle dont la partie alkyle linéaire ou ramifiée est en C₁ - C₆,
X est un hétéroatome choisi parmi O et S.
R5 est un radical capteur d'électrons choisi parmi un radical cyano, carbamoyle, phényloxycarbonyle, alcoxycarbonyle et phénylcarbonyle dans lequel la partie alkyle linéaire, cyclique ou ramifiée est en C₁ - C₆,
R6 est un radical donneur d'électron choisi parmi un radical alkylthyoéther, phénylthioéther, amino, alkylamino, alcoxy et phénoxy dans lequel la partie alkyle linéaire, cyclique ou ramifiée est en C₁ - C₆.

2. Procédé selon la revendication 1, caractérisé en ce que ledit monomère est choisi parmi le styrène, le butadiène, les esters (métha)cryliques et les nitriles vinyliques.

3. Procédé selon la revendication 2, caractérisé en ce qu'on polymérise au moins 60 % en poids dudit monomère avec jusqu'à 40 % en poids d'autres monomères insaturés copolymérisables choisis parmi :
- les esters vinyliques d'acide carboxylique,
- les acides mono- et dicarboxyliques à insaturation éthylénique,
- les amides d'acides carboxyliques insaturés,
- les monomères éthyléniques comportant un groupe acide sulfonique, et
- les esters des acides (meth)acryliques avec les alcanediols.

4. Procédé selon l'une quelconque des revendications 1 à 3 caractérisé en ce qu'on utilise entre 0,05 et 10 %, de préférence entre 0,1 et 3 % en poids d'agent de transfert par rapport au poids total des monomères.

5. Procédé selon l'une quelconque des revendications 1 à 4, caractérisé en ce que la polymérisation est éffectuée en masse,en solution dans un suivant organique ou en émulsion aqueuse.

6. Polymère obtenu selon un procédé tel que défini à l'une quelconque des revendications 1 à 5 et fonctionnalisé en bout de chaîne par un diène conjugué provenant de l'agent de transfert.

7. Diène, caractérisé en ce qu'il répond à la formule :

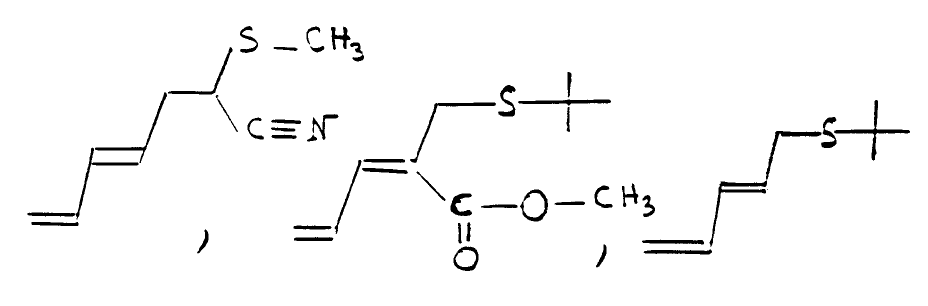

## Claims

1. Process for the preparation of polymers according to which at least one monomer containing ethylenic unsaturation is polymerized by radical polymerization in the presence of an effective amount of a conjugated diene transfer agent corresponding to the general formula: 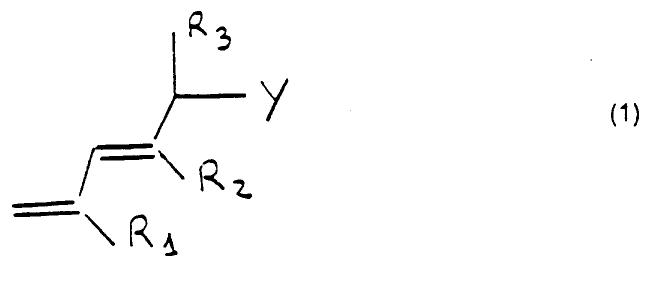 in which:
the radicals R₁, R₂ and R₃, which are identical or different, are chosen from a hydrogen atom, a linear or branched C₁-C₆ alkyl radical, a phenyl radical, an alkoxycarbonyl radical, an acyloxy radical and an acylamino radical, in which the alkyl part is linear or branched C₁-C₆, a phenoxycarbonyl radical and a cyano radical,
the radical Y is chosen from the radicals:
X-R₄ and -CH(R₅R₆) in which:
R₄ is chosen from a linear or branched C₁-C₆ alkyl radical, a phenyl radical and an acyl radical in which the linear or branched alkyl part is C₁-C₆,
X is a heteroatom chosen from O and S,
R₅ is an electron-withdrawing radical chosen from a cyano, carbamoyl, phenyloxycarbonyl, alkoxycarbonyl and phenylcarbonyl radical in which the linear, cyclic or branched alkyl part is C₁-C₆,
R₆ is an electron-donating radical chosen from an alkylthio ether, phenylthio ether, amino, alkylamino, alkoxy and phenoxy radical in which the linear, cyclic or branched alkyl part is C₁-C₆.

2. Process according to claim 1, characterized in that the said monomer is chosen from styrene, butadiene, (meth)acrylic esters and vinyl nitriles.

3. Process according to claim 2, characterized in that at least 60 % by weight of the said monomer is polymerized with up to 40 % by weight of other copolymerizable unsaturated monomers chosen from:
- carboxylic acid vinyl esters,
- mono- and dicarboxylic acids containing ethylenic unsaturation,
- unsaturated carboxylic acid amides,
- ethylene monomers containing a sulphonic acid group, and
- esters of (meth)acrylic acids with alkanediols.

4. Process according to any one of claims 1 to 3, characterized in that use is made of between 0.05 and 10 %, preferably between 0.1 and 3 %, by weight of transfer agent with respect to the total weight of the monomers.

5. Process according to any one of claims 1 to 4, characterized in that the polymerization is carried out in bulk, in solution in an organic solvent or in an aqueous emulsion.

6. Polymer obtained according to a process as defined in any one of claims 1 to 5 and functionalized at the chain end by a conjugated diene originating from the transfer agent.

7. Diene, characterized in that it corresponds to the formula:

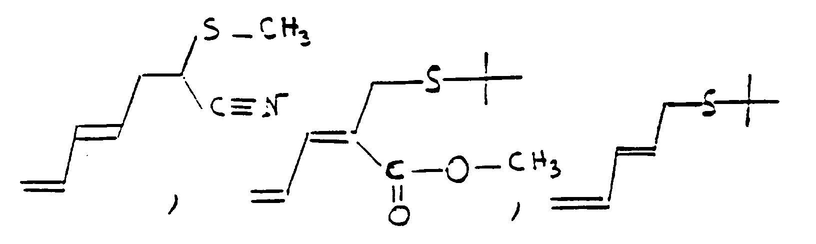

## Patentansprüche

1. Verfahren zur Herstellung von Polymeren, wobei man mindestens ein ethylenisch ungesättigtes Monomeres in Gegenwart einer wirksamen Menge eines Transfermittels eines konjugierten Diens der allgemeinen Formel 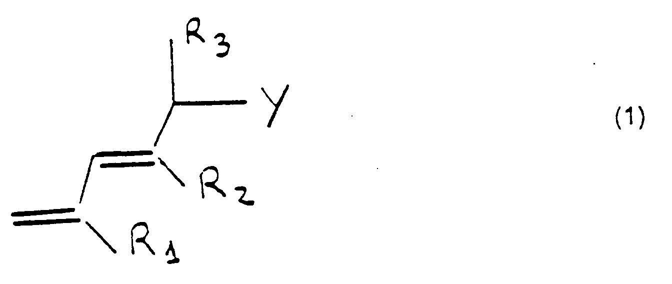 worin die Reste R₁, R₂ und R₃, die gleich oder verschieden sind, ausgewählt sind aus einem Wasserstoffatom, einem linearen oder verzweigten C₁-C₆-Alkylrest, einem Phenylrest, einem Alkoxycarbonylrest, einem Acyloxyrest und einem Acylaminorest, in denen der Alkylteil ein linearer oder verzweigter C₁-C₆-Rest ist, einem Phenoxycarbonylrest und einem Cyanorest,
der Rest Y ausgewählt ist aus den Resten
X-R4 und -CH(R5 R6), worin
R4 aus einem linearen oder verzweigten C₁-C₆-Alkylrest, einem Phenylrest und einem Acylrest, deren Alkylteil ein linearer oder verzweigter C₁-C₆-Rest ist, ausgewählt ist, X ein Heteroatom, ausgewählt aus O und S, ist,
R5 ein elektroneneinfangender Rest ist, ausgewählt aus einem Cyano-, Carbamoyl-, Phenyloxycarbonyl-, Alkoxycarbonyl- und Phenylcarbonylrest, worin der Alkylteil ein linearer, zyklischer oder verzweigter C₁-C₆-Rest ist, R6 ein elektronenspendender Rest ist, ausgewählt aus einem Alkylthioether-, Phenylthioether-, Amino-, Alkylamino-, Alkoxy- und Phenoxyrest, worin der Alkylteil ein linearer, zyklischer oder verzweigter C₁-C₆-Rest ist, radikalisch polymerisiert.

2. Verfahren nach Anspruch 1, dadurch **gekennzeichnet**, daß man das Monomere aus Styrol, Butadien, (Meth)acrylestern und Vinylnitrilen auswählt.

3. Verfahren nach Anspruch 2, dadurch **gekennzeichnet**, daß man mindestens 60 Gew.-% des Monomeren mit bis zu 40 Gew.-% an anderen ungesättigten copolymerisierbaren Monomeren, ausgewählt aus
- Carbonsäurevinylestern,
- ethylenisch ungesättigten Mono- und Dicarbonsäuren,
- ungesättigten Carbonsäureamiden,
- Ethylenmonomeren mit einer Sulfonsäuregruppe und
- Alkandiol(meth)acrylsäureestern, polymerisiert.

4. Verfahren nach einem der Ansprüche 1 bis 3, dadurch **gekennzeichnet**, daß man zwischen 0,05 und 10 Gew.-%, bevorzugt zwischen 0,1 und 3 Gew.-%, des Transfermittels, bezogen auf das Gesamtgewicht der Monomeren, verwendet.

5. Verfahren nach einem der Ansprüche 1 bis 4, dadurch **gekennzeichnet**, daß man die Polymerisation in der Masse, in Lösung in einem organischen Lösungsmittel oder in einer wäßrigen Emulsion durchführt.

6. Polymeres, erhalten nach einem Verfahren nach einem der Ansprüche 1 bis 5 und derivatisiert am Kettenende durch ein konjugiertes Dien, das aus dem Transfermittel stammt.

7. Dien, dadurch **gekennzeichnet**, daß es der Formel 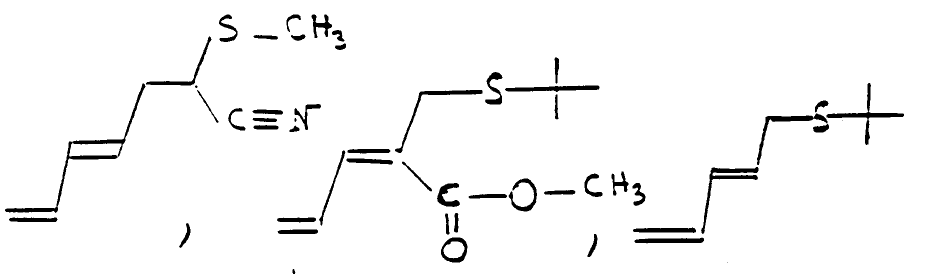 entspricht.
